(19) Europäisches Patentamt
European Patent Office
Office européen des brevets

(11) Veröffentlichungsnummer: **0 079 020 B1**

(12) **EUROPÄISCHE PATENTSCHRIFT**

(45) Veröffentlichungstag der Patentschrift: **05.02.86**

(51) Int. Cl.[4]: **C 07 D 233/76**, C 08 G 73/10, C 09 D 3/49

(21) Anmeldenummer: **82110065.8**

(22) Anmeldetag: **02.11.82**

(54) **Verfahren zur Herstellung von Hydantoinen.**

(30) Priorität: **11.11.81 DE 3144698**
**11.11.81 DE 3144697**

(43) Veröffentlichungstag der Anmeldung:
**18.05.83 Patentblatt 83/20**

(45) Bekanntmachung des Hinweises auf die Patenterteilung:
**05.02.86 Patentblatt 86/6**

(84) Benannte Vertragsstaaten:
**AT DE FR GB IT**

(56) Entgegenhaltungen:
**EP - A - 0 002 662**
**EP - A - 0 033 477**
**US - A - 3 254 036**

(73) Patentinhaber: **BAYER AG, Konzernverwaltung RP Patentabteilung, D-5090 Leverkusen 1 Bayerwerk (DE)**

(72) Erfinder: **Schulte, Bernhard, Dr., Suedwall 80 A, D-4150 Krefeld (DE)**
Erfinder: **Jakob, Wolfgang, Am Domacker 81, D-4130 Moers 1 (DE)**
Erfinder: **Dünwald, Willi, Dr., Geschwister-Scholl-Strasse 16, D-5090 Leverkusen 1 (DE)**
Erfinder: **Meyer, Karl-Heinrich, Dr., Deswatinesstrasse 89, D-4150 Krefeld (DE)**
Erfinder: **Zecher, Wilfried, Dr., Treptower Strasse 6, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 079 020 B1

LIBER, STOCKHOLM 1986

**Beschreibung**

Die Erfindung betrifft ein Verfahren zur Herstellung von Verbindungen mit mindestens einem Hydantoinring im Molekül durch Umsetzung von Carbodiimiden mit Maleinsäureanhydrid oder substituierten Bernsteinsäureanhydriden und mono- oder polyfunktionellen aromatischen Alkoholen.

Hydantoine, Polyhydantoine und Verfahren zu ihrer Herstellung sind bekannt (vergl. z. B. Am. Chem. J. 45, 383; BE-PS 678 282H; EP-A-0002662 und EP-A-0033477).

Monomolekulare Hydantoine können im Pharma- und Pflanzenschutzbereich verwendet werden, und Polyhydantoine haben als temperaturbeständige Beschichtungsmittel auf dem Elektroisolierlacksektor Bedeutung erlangt (FR-PS 1 484 694).

Gegenstand der Erfindung ist ein neues Verfahren zur Herstellung von Hydantoinringe enthaltenden Verbindungen, das dadurch gekennzeichnet ist, daß man ein Carbodiimid der Formeln I oder II

$$R^1 - N = C = N - R^2 \qquad [-Y - N = C = N-]_n$$

(I) (II)

in denen

$R^1$ und $R^2$ gleich oder verschieden einen aliphatischen Rest mit 1 - 20 C-Atomen, einen cycloaliphatischen Rest mit 5 - 12 C-Atomen, einen aliphatisch-aromatischen Rest mit 6 - 20 C-Atomen, einen aromatischen Rest mit 6 - 16 C-Atomen, einen Heteroatome wie N, 0 oder S enthaltenden aromatischen oder cycloaliphatischen Rest mit 5 - 12 C-Atomen, die jeweils gegebenenfalls mit Halogen (vorzugsweise Chlor, Brom, Jod, Fluor) Nitril-, $C_2$-$C_{12}$-Dialkyl-amino-, $C_7$-$C_{12}$-Alkylarylamino-, $C_2$-$C_{18}$-Alkoxycarbonyl C -$C_{18}$ . Aroxycarbonyl-, $C_2$-$C_{18}$-Alkylcarboxy-, $C_7$-$C_{18}$Arylcarboxy-, $C_1$-$C_{18}$-Alkoxy-, $C_6$-$C_{18}$-Aroxy-, $C_1$-$C_{18}$-Alkyl- bzw. Halogenalkyl- oder Nitrogruppen substituiert sein können, oder einen $C_2$-$C_{12}$-Dialkylamino-, $C_2$-$C_{10}$-Alkoxycarbonyl-, $C_6$-$C_{18}$-Glykosylrest oder eine -$Si(R^3)_3$-, -Sn $(R^3)_3$-, -SO $R^3$-Gruppe (mit $R^3$ = $C_6$-$C_{12}$-Aryl bzw. $C_1$-$C_8$-Alkyl) bedeuten oder miteinander als Glieder entsprechend cyclischer organischer Reste verknüpft sein können, und

die für $R^1$, $R^2$ angegebene Bedeutung hat und dabei bevorzugt für aliphatische Reste mit 2 - 12 C-Atomen, cycloaliphatische Reste mit 5 - 12 C-Atomen, $C_6$-$C_{16}$-Arylreste oder über 0, S' $SO_2$ $CH_2$, $CH_3$-C-$CH_3$ oder CO verknüpfte Diphenylreste oder für -$Si(R^3)_2$-, -$Sn(R^3)_2$-Gruppen steht, und

eine ganze Zahl von 2 - 2000, vorzugsweise 2 bis 1000 bedeutet, mit Maleinsäureanhydrid oder mit Derivaten des Bernsteinsäureanhydrids der Formel

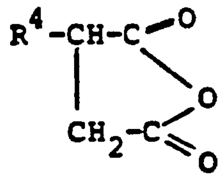

(III)

wobei $R^4$ Halogen, eine Alkylcarboxygruppe oder Arylcarboxygruppe bedeutet, in Gegenwart von mono- oder mehrfunktionellen aromatischen OH-funktionellen Verbindungen (wie z.B. Phenol, Kresol, 2,2-Bis-(4-hydroxyphenyl)-propan) bei Temperaturen von ca. 20 bis ca. 250°C, gegebenenfalls in Anwesenheit eines Katalysators und gegebenenfalls in Anwesenheit eines Lösungsmittels umsetzt.

Es ist bekannt, daß Maleinsäure durch Carbodiimide in Maleinsäureanhydrid überführt wird ( J. Prakt. Chem. 79, 513 (1909)) und daß organische Carbodiimide zur Entfernung von Säurespuren aus den entsprechenden Anhydriden eingesetzt werden können (Chem. Abstr. 58, 9254 (1963)), das heißt, Carbodiimide können gegenüber Säureanhydriden als inert betrachtet werden. Erst bei hohen Reaktionstemperäturen, z.B. in siedendem Dimethylformamid wurde bei der Reaktion von Diphenylcarbodiimid mit Säureanhydriden die Bildung der N-Acyl-N,N'-disubstituierten Harnstoffe festgestellt (Chem. Rev. 67, 2 (1967) S. 107; J. Am. Chem. Soc. 21, 136 (1899); Chem. Ber. 89, 2681 (1956) ). Hydantoine werden so nicht gebildet.

Überraschenderweise gelingt die Hydantoinbildung, wenn man nach den erfindungsgemäßen Bedingungen arbeitet und Carbodiimide mit Maleinsäureanhydrid oder substituierten Bernsteinsäureanhydriden in Gegenwart von monooder mehrfunktionellen aromatischen Alkoholen zur Reaktion bringt.

Besonders vorteilhaft gelingt nach dem erfindungsgemäßen Verfahren die Herstellung von Polyhydantoinen, wobei der aromatische Alkohol sowohl als Reaktionspartner als auch als Lösungsmittel für das polymere Reaktionsprodukt dienen kann, und Polymere mit sehr hohen Erweichungstemperaturen erhalten werden, die als hochtemperaturbeständige Beschichtungsmittel, insbesondere auf dem Sektor der Elektroisolierlacktechnik, geeignet sind.

Als Carbodiimid-Verbindungen im Sinne der Erfindung können Monocarbodiimide mit einer -N=C=N-Gruppe im Molekül, deren cyclische Dimere oder Trimere oder auch lineare bzw. verzweigte Polycarbodiimide mit mehr als zwei CarbodiimidGruppen im Molekül verwendet werden. Als solche kommen in Betracht:

Carbodiimide der Formeln I und II

$$R^1 - N = C = N - R^2 \qquad [-Y - N = C = N-]_n$$

(I) (II)

in denen

$R^1$ und $R^2$ gleich oder verschieden einen

aliphatischen Rest mit 1 - 20 C-Atomen, einen cycloaliphatischen Rest mit 5 - 12 C-Atomen, einen aliphatisch-aromatischen Rest mit 6 - 20 C-Atomen, einen aromatischen Rest mit 6 - 16 C-Atomen, einen Heteroatome wie N, O oder S enthaltenden aromatischen oder cycloaliphatischen Rest mit 5 - 12 C-Atomen, die jeweils gegebenenfalls mit Halogen (vorzugsweise Chlor, Brom, Jod, Fluor), Nitril-, $C_2$-$C_{12}$-Dialkyl-amino-, $C_7$-$C_{12}$-Alkylarylamino-, $C_2$-$C_{18}$-Alkoxycarbonyl-, $C_7$-$C_{18}$Aroxycarbonyl-, $C_2$-$C_{18}$-Alkylcarboxy-, $C_7$-$C_{18}$Arylcarboxy-, $C_1$-$C_{18}$-Alkoxy-, $C_6$-$C_{18}$-Aroxy-,$C_1$-$C_{18}$-Alkyl- bzw. Halogenalkyl- oder Nitrogruppen substituiert sein können, oder einen $C_2$-$C_{12}$-Dialkylamino-, $C_2$-$C_{10}$-Alkoxycarbonyl-, $C_6$-$C_{18}$-Glykosylrest oder eine -$Si(R^3)_3$-, -$Sn(R^3)_3$-, -$SO_2R^3$-Gruppe (mit $R^3$ = $C_6$-$C_{12}$-Aryl bzw. $C_1$-$C_8$-Alkyl) bedeuten oder miteinander als Glieder entsprechend cyclischer organischer Reste verknüpft sein können, und

Y die für $R^1$, $R^2$ angegebene Bedeutung hat und dabei bevorzugt für aliphatische Reste mit 2 - 12 C-Atomen, cycloaliphatische Reste mit 5 - 12 C-Atomen, $C_6$-$C_{16}$-Arylreste oder über 0, S' $SO_2$ $CH_2$, $CH_3$-C-$CH_3$ oder CO verknüpfte Diphenylreste oder für -$Si(R^3)_2$-, -$Sn(R^3)_2$-Gruppen steht, und

n eine ganze Zahl von 2 - 2000, vorzugsweise 2 bis 1000 bedeutet.

Als Monocarbodiimide werden erfindungsgemäß N,N'- symmetrisch und/oder asymmetrisch substituierte aliphatische, aliphatisch-aromatische, cyclische, heterocyclische, aromatische, gegebenenfalls durch Heteroatome substituierte Verbindungen mit einer -N=C=N-Gruppe im Molekül eingesetzt, z. B. Dialkylcarbodiimide wie Dimethyl-, Diethyl-, Diisopropyl-, Dihexyl-, Dibutyl-, Dinonyl, Didodecyl-, und Distearylcarbodiimid, vorzugsweise aromatische, gegebenenfalls substituierte Monocarbodiimide wie Diphenyl-, Ditolyl-, Dinaphthylcarbodiimid, Di-(p-iodphenyl)-, Di(p-dimethylaminophenyl)-, Dipyridyl-carbodiimids, Di-Nitro-, -Alkoxy-, -Aroxy-, -Chlor-, -Dichlor-, -Trichlor-, -Tetrachlor-, -Pentachlorphenyl, -Benzyl-, -p-Bromphenylcarbodiimid oder Carbodiimid-dibenzoesäureester, -di-phthalsäureester, -di-isophthalsäureester, Carbodiimid-dibenzonitril, cycloaliPhatische Carbodiimide wie Dicyclohexylcarbodiimid und ungesättigte Carbodiimide wie Diallyl-, Dioleyl-, Dicyclohexenylcarbodiimid.

Diese Carbodiimid-Verbindungen können nach bekannten Verfahren z.B. aus den entsprechenden Thioharnstoffen in Gegenwart von Metalloxiden, Quecksilbersalzen, Natriumsalzen, Arylsulfochloriden bzw. durch Oxidation von Thioharnstoffen oder aus S-Alkylisothioharnstoffen, HarnstoffVerbindungen, wie z.B. in Chem. Rev. 67,2 (1967), S. 107 angegeben oder aus den entsPrechenden Isocyanat-Verbindungen unter Kohlendioxidabspaltung in Gegenwart der bekannten speziellen Katalysatoren zur $CO_2$-Abspaltung hergestellt werden (FR-PS 1 180 307).

Weiterhin können die N-Sulfonylcarbodiimide $RSO_2N=C=NR$, die N-Aminocarbodiimide $RN=C=N-NR_2$ oder die N,N'-Disilylcarbodiimide, wie diese z.B. in Chem. Rev. 67, 2 (1967), S. 107; Angew. Chem. 77, 430 (1965); J. Org. Chem. 29, 2816 (1964); Ann. 652, 21 (1962); Z. Anorg. Allgem. Chem. 330, 101 (1964) aufgeführt sind, eingesetzt werden.

Als erfindungsgemäß einzusetzende Ausgangskomponenten kommen ebenso aliphatische, cycloaliphatische, araliphatische, aromatische und heterocyclische lineare oder verzweigte Polycarbodiimide mit mehr als 2 Carbodiimidgruppen in Betracht, sowie deren Gemische oder Polycarbodiimide, die eine statistische Zusammensetzung oder einen blockartigen Aufbau aus unterschiedlichen Strukturelementen in bestimmter Sequenzlänge im Polymermolekül aufweisen und somit die oben angeführten aliphatischen, cycloaliphatischen, araliphatischen, aromatischen und heterocyclischen Struktureinheiten in unterschiedlichsten Verhältnissen sowohl statistisch verteilt als auch blockweise angeordnet im Polymermolekül enthalten können.

Im Falle der Synthese dieser Polycarbodiimide mit zwei oder mehr Carbodiimidgruppen im Molekül aus mehrfunktionellen Isocyanaten können die aus der Literatur bekannten Katalysatoren (vergl. z.B. FR-PS 1 180 307), beispielsweise Phospholine, Phospholidinsulfide oder auch metallorganische Verbindungen der Gruppe Ia - IIIa, wie z.B. Phenyllithium, Diethylzink eingesetzt werden.

Dabei können die erfindungsgemäßen Polycarbodiimid-Verbindungen aus Polyisocyanaten, wie sie z.B. in Annalen 562, S. 75 - 136; Am. Chem. J. 45, 383; DE-OS 27 14 655; USPS 3 397 253; EP-PS 0012379 umfassend aufgeführt sind, hergestellt werden.

Besonders bevorzugt sind Gemische aus Poly-Toluylencarbodiimiden (2,4- und 2,6-Substitutionsprodukte), Polyp- und m-phenylencarbodiimiden, sowie Polycarbodiimide auf der Basis von Anilin-Formaldehydkondensaten mit Polyphenylenmethylen-Struktur und Poly-4,4'-Diphenylether-, Polyp-Phenylen-, Poly-Naphthylencarbodiimid, Polyisophoron-carbodiimid, Polyhexamethylencarbodiimid, Polycumencarbodiimid, Polymesitylencarbodiimid und/oder deren Gemische, sowie Blockpolycarbodiimide z.B. der folgenden Strukturen:

-B-B-B-A-A-A-A-B-B-B

-C-C-B-B-B-A-A-A-A-B-B-B-C-C

wobei A z.B. ein aromatisches Strukturelement wieDiphenylmethan ist, B einen aliphatischen Rest R wie z.B. den Isophoronrest und C eine aromatische Einheit wie z.B. die Toluylen- oder Naphthylengruppe darstellt. Diese Blockpolycarbodiimide können z.B. hergestellt werden, indem die Carbodiimidisierung der einzelnen verwendeten mehrfunktionellen

Isocyanate stufenweise nacheinander erfolgt. Die aufgeführten Strukturen und technisch leicht zugänglichen bisfunktionellen Isocyanate verdeutlichen die Variationsbreite im Hinblick auf Sequenzlängen und Mengenverhältnisse der einzelnen Bausteine, wobei die Polycarbodiimide auch gezielt verzweigt werden können, wenn z.B. tri- und mehrfunktionelle Isocyanate in den Carbodiimidisierungsstufen eingesetzt werden. Als erfindungsgemäß einzusetzende Anhydridverbindungen sind substituierte Bernsteinsäureanhydride der allgemeinen Formel III

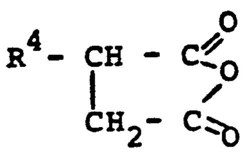

(III)

worin

$R^4$ Halogen wie Chlor, Brom, Iod, Fluor (vorzugsweise Chlor) oder die R'-COO-Gruppe mit R' = Alkyl mit 1 - 6 C-Atomen bzw. Aryl mit 6 - 10 C-Atomen bedeutet, geeignet (bevorzugt sind Chlorbernsteinsäureanhydrid und Acetoxybernsteinsäureanhydrid, die z.B. durch Reaktion von Äpfelsäure mit Thionylchlorid bzw. Acetylchlorid hergestellt werden können) oder

besonders bevorzugt wird Maleinsäureanhydrid verwendet.

Geeignete aromatische OH-funktionelle Verbindungen entsprechen der allgemeinen Formel IV

$R^5 - (OH)_m$ (IV),

in der

$R^5$ einen aromatischen Rest mit $C_6$-$C_{20}$ C-Atomen, der gegebenenfalls einfach oder mehrfach mit Halogen, vorzugsweise Chlor, Brom, Iod, Fluor, $C_1$-$C_8$-Alkyl-, $C_6C_{12}$-Aryl-, $C_1$-$C_8$-Halogenalkyl-, $C_1$-$C_8$-Alkoxy-, $C_6$-$C_{12}$Aroxy-, $C_1$-$C_8$-Alkoxycarbonyl-, $C_7$-$C_{15}$-Aroxycarbonyl-, $C_2$-$C_8$-Alkylcarboxy-, $C_7$-$C_{17}$-Arylcarboxy-, $C_1$-$C_6$-Alkylamino-, $C_2$-$C_{12}$-Dialkylamino-, Amino- oder Nitrogruppen substituiert sein kann, bedeutet oder für über 0, S, $SO_2$, CH, $CH_2$, $CH_3$-C-$CH_3$, CO, -N=N-, Cyclohexyl miteinander verknüpfte Phenylreste oder den Isatinbiskresylbzw. IsatinbisPhenylrest steht und

m eine ganze Zahl von 1 - 4, vorzugsweise 1 - 2, besonders bevorzugt 1 bedeutet.

$R^5$ leitet sich vorzugsweise von Benzol, Toluol, Xylol, Azobenzol, Naphthalin, Anthracen, Triphenylmethan, Diphenylmethan, Diphenylsulfon, Diphenoxy, Diphenylsulfid, Diphenylisopropan, Diphenylcyclohexan ab. Besonders bevorzugt sind Phenole, Kresole, Xylenole und deren Gemische.

Die erfindungsgemäße Umsetzung läßt sich beispielhaft durch folgende Gleichungen A bzw. B wiedergeben.

**A. Bei Verwendung von Maleinsäureanhydrid**

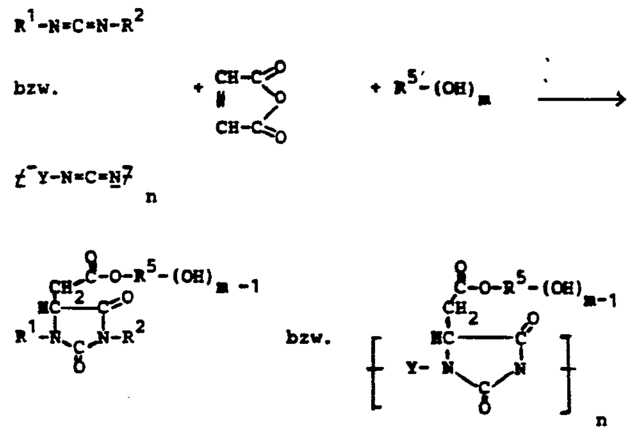

**B. Bei Verwendung von substituierten Bernsteinsäureanhydriden**

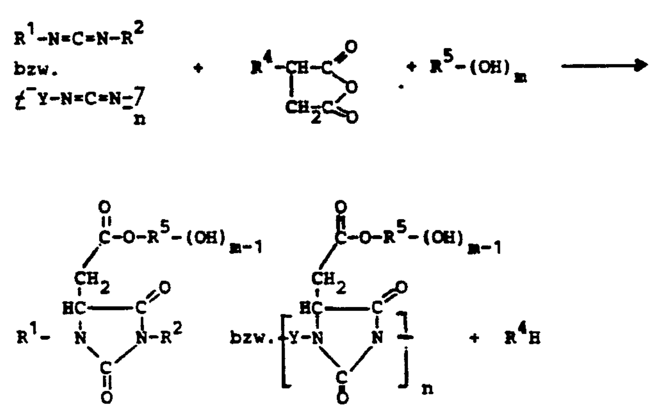

Im allgemeinen werden dementsprechend pro Äquivalent Carbodiimid mindestens 1 Val des Anhydrids und ebenso mindestens 1 Val eines monofunktionellen aromatischen Alkohols wie

z.B. Phenol oder Kresol eingesetzt - bei angestrebter Verzweigung z.B. des Polymermoleküls wird die Funktionalität der Hydroxyverbindung mit m - 1 entsprechend berücksichtigt. Doch sind auch sehr weitgehende Abweichungen Von diesen Mengenverhältnissen möglich.

Die erfindungsgemäßen, in 5-Stellung substituierten Hydantoine können eindeutig IR-spektroskopisch anhand der typischen Banden für Hydantoine und aromatische Estercarbonylgruppen und mit Hilfe der NMR-Spektroskopie identifiziert werden. Die polymeren Produkte besitzen Lösungsviskositäten von 200 bis 200.000 mPas, vorzugsweise von 1.000 bis 100.000 mPas, die in 30 gew.-%igen Lösungen mit beispielsweise Kresol, Butyrolacton, Acetophenonen, Benzoesäurealkylestern, Benzylalkohol bei 25° C bestimmt werden.

Die erfindungsgemäße Umsetzung kann in Lösungsmitteln, die unter den Reaktionsbedingungen nicht reagieren oder gegebenenfalls lockere, weiterreagierende Additionsverbindungen bilden, oder als heterogene Reaktion, d.h., in Gegenwart von Verdünnungsmitteln in Suspension oder in Substanz oder in einem Überschuß einer der Reaktionskomponenten durchgeführt werden. Bevorzugt wird die Hydroxyverbindung, wie z.B. Kresol, Phenol, Xylenol, im Überschuß eingesetzt.

Als Reaktionsmedien eignen sich inerte organische Flüssigkeiten, z.B. aliphatische, aromatische Kohlenwasserstoffe, Halogenkohlenwasserstoffe, Heterocyclen, Ester, Lactone, Ketone, Sulfoxide, Sulfone, Ether, substituierte Amide, Nitrile, Phosphorsäureamide. Beispiele dafür sind Cyclohexan, Ligroine, Tetrachlorkohlenstoff, Chloroform, Methylenchlorid, Ethylenchlorid, Tetrachlorethan, Methylethylketon, Diisopropylether, Ethylenglykol-, Diethylenglykoldimethyl- bzw. -diethylether, Dioxan, Tetrahydrofuran, Toluol, Xylole, Chlorbenzol, Dichlorbenzol, Acetophenon, Cyclohexanon, propylencarbonat, Caprolactam, Caprolacton, Butyrolacton, Glykolmonomethyletheracetat, Dimethylsulfoxid, Tetramethylsulfon, Benzoesäurealkylester, N-Methylpyrrolidon, Dimethylformamid, Dimethylacetamid, Hexamethylphosphorsäuretriamid, Benzonitril und deren Gemische.

Aromatische Hydroxyverbindungen wie phenole, Kresole, Xylenole, die erfindungsgemäß sowohl als Reaktionspartner als auch als Lösungsmittel fungieren können, werden mit Vorteil verwendet.

Bevorzugt werden für die Herstellung von Monohydantoinen Kombinationen aus niedrig- und höhersiedenden Flüssigkeiten, z.B. Gemische aus Methylenchlorid/Chlorbenzol bzw. o-Dichlorbenzol oder Methylenchlorid/Xylol bzw. Toluol bzw. phenol verwendet.

Für die Synthese der polymeren Reaktionsprodukte werden insbesondere ternäre Gemische aus den aufgeführten Lösungs- bzw. Verdünnungsmitteln eingesetzt, wie z.B. Methylenchlorid/Toluol bzw. Xylol/ -Butyrolacton bzw. phenol bzw. Kresol. Dabei ist die am leichtesten flüchtige Komponente zur Wärmeabfuhr durch Abdestillieren oder als gutes Lösungsmittel für die polycarbodiimide geeignet. Die höhersiedende Verbindung kann nach Abschluß der Reaktion zum Polyhydantoin noch teilweise oder vollständig in dem Reaktionsgemisch verbleiben, wie z.B. Toluol, Xylol, Solvesso. Diese Komponenten sind somit bevorzugt in der Elektroisolierlacktechnik gebräuchliche Verschnittmittel. Die hochsiedende Komponente wie Butyrolacton, phenol usw. stellt das eigentliche Lösungsmittel für das Reaktionsprodukt dar.

Als Verschnitt- bzw. Verdünnungsmittel können außerdem Cyclohexan, Solventnaphtha oder nach erfolgter Umsetzung des Carbodiimids mit dem Anhydrid und dem aromatischen Alkohol auch Hydroxyalkylether oder aliphatische, aliphatisch-aromatische Alkohole, Butanol, Aminoalkohol, Benzylalkohol, phenoxyethanol, die Methyl-, Ethyl-, Isopropyl-, Butylmonoether des Ethylen-, Diethylen- bzw. Propylenglykols zugesetzt werden, die dann die Herstellung von gegebenenfalls umweltfreundlichen, nichtphenolischen Polymerlösungen ermöglichen.

Die erfindungsgemäße Umsetzung kann in Gegenwart von Katalysatoren, die die Addition von OH-funktionellen Verbindungen an die Carbodiimidgruppe beschleunigen, wie z.B. Kupfer-I-, Kupfer-II-chlorid usw. oder auch unter Zusatz der für die Malein-Fumarsäureumlagerung geeigneten Katalysatoren, wie z.B. Iod usw. oder unter Verwendung der für die Cyclisierung zum Hydantoinring bekannterweise geeigneten Katalysatoren wie Basen, beispielsweise Triethylamin, N-Methylmorpholin, Endoethylenpiperazin, wie Säuren, z.B. Essigsäure, p-Toluolsulfonsäure, wie Metalle, insbesondere von Eisen, Blei, Zink, Zinn, Kupfer, Kobalt, Titan, Mangan, beispielsweise Titantetrabutylat, Titanaminoalkohol, Eisenacetylacetonat, Dibutylzinnlaurat, Bleiacetat, Bleinaphthenat, Bleiethylhexoat, Zinnoctoat oder Calciumnaphthenat durchgeführt werden.

Insbesondere geeignet sind Iod, Endoethylenpiperazin (Diazabicyclooctan) und Katalysatoren, die zur Bildung der Carbodiimide benutzt werden, z.B. phospholinoxid.

In den meisten Fällen kann die Reaktion auch ohne Katalysator-Zusatz der oben beschriebenen Art durchgeführt werden.

Im Falle der Verwendung von Carbodiimiden, die aus Isocyanaten in bekannter Weise (vergl. z.B. DE-AS 1122 057; US-pS 2 941 983) hergestellt worden sind, kann direkt das Reaktionsgemisch einschließlich des gegebenenfalls darin enthaltenen Katalysators benutzt werden.

Zur Durchführung der erfindungsgemäßen Verfahren werden die Carbodiimide, vorzugsweise gelöst oder suspendiert, mit Maleinsäureanhydrid oder den substituierten

Bernsteinsäureanhydriden in Gegenwart der aromatischen Hydroxyverbindung einige Minuten bis zu mehreren Stunden bei Temperaturen von ca. 20°C bis ca. 250°C, bevorzugt 30°C bis 200°C, gehalten.

Der Reaktionsverlauf kann IR-spektroskopisch verfolgt werden.

Die drei Reaktionskomponenten werden vorzugsweise bei niedrigen Temperaturen von 20°C bis 50°C, gegebenenfalls unter Kühlung, vereinigt oder einer der Reaktanten wird portionsweise eingetragen, vorzugsweise bei ca. 20°C bis 45°C. Dabei können sowohl die Carbodiimide als Lösungen oder Suspensionen oder die Anhydridverbindungen als auch der aromatische Alkohol, gegebenenfalls gemeinsam mit dem Anhydrid, mit oder ohne weitere Lösungsmittel vorgelegt werden.

Vorteilhafterweise werden im Hinblick auf eine technische Polyhydantoinherstellung im Falle der Carbodiimidisierung Von mehrfunktionellen Isocyanaten die Carbodiimide vorgelegt, da sie nach Abschluß ihrer Herstellung sofort weiter zum erfindungsgemäßen Hydantoin umgesetzt werden können.

Werden polycarbodiimide eingesetzt, die einen blockartigen Aufbau -B-A-B- aufweisen, wobei A und B Sequenzen von jeweils verschiedenen Kettengliedern darstellen, wird im allgemeinen so verfahren, daß ein Diisocyanat (z.B. 4,4'-Diisocyanatodiphenylmethan) in den aufgeführten, für die Herstellung von Monohydantoinen geeigneten Reaktionsmedien aus einer leicht- und schwerer flüchtigen Flüssigkeit, wie z.B. Methylenchlorid/Toluol, bis zum nahezu vollständigen Umsatz carbodiimidisiert wird. Anschließend wird, gegebenenfalls unter Zusatz von weiterem Lösungs-/Verdünnungsmittel, das Diisocyanat - oder auch z.B. Triisocyanat - mit der Struktureinheit B (z.B. Isophoron-, Tolyl-) zugegeben und die Carbodiimidisierung fortgesetzt, wobei dann mit Vorteil Monoisocyanate (z.B. phenyl-, Tolyl-, Naphthyl-, Cyclohexyl-, Methyl-, Cyclohexenyl-, Oleylisocyanat, Isocyanatobenzoesäureester, -phthalsäureester, -isophthalsäureester usw.) im Verlauf des weiteren Polycarbodiimidaufbaues als Regler eingetragen werden.

Anschließend können die Anhydride und aromatischen Hydroxyverbindungen, indem sie gegebenenfalls gleichzeitig oder nacheinander oder gemeinsam gelöst in den angegebenen geeigneten Reaktionsmedien zugesetzt werden, zur Reaktion gebracht.werden.

Bei Einsatz von Phenolen, Kresolen, Xylenolen wird vorteilhafterweise so verfahren, daß dem vorgelegten Carbodiimid zunächst die stöchiometrisch erforderliche Menge oder ein Überschuß zugesetzt wird, anschließend das Anhydrid portionsweise eingetragen und im Verlauf der Reaktion weiteres Phenol usw. zugegeben wird, wenn diese Reaktionskomponente auch als Lösungsmittel dienen soll.

Dabei wird der Reaktionsverlauf zum Hydantoin mit Vorteil durch stufenweise Steigerung der Reaktionstemperatur erreicht. Es können Inhibitoren, wie z.B. Toluhydrochinon zugesetzt werden, um eine eventuell einsetzende Polymerisation der Doppelbindung zu vermeiden.

Die Umsetzung wird zweckmäßigerweise unter einem inerten Schutzgas wie $N_2$ oder Argon durchgeführt.

Die erfindungsgemäße Reaktion kann kontinuierlich oder diskontinuierlich bei Normaldruck oder bei Überdruck ausgeführt werden.

Die Aufarbeitung der niedermolekularen Reaktionsprodukte kann nach gängigen Methoden wie z.B. Kristallisation oder Umfällen erfolgen.

Die nach dem erfindungsgemäßen Verfahren zugänglichen monomolekularen Hydantoine zeigen Wirkungen auf dem pharmazeutischen und Pflanzenschutzsektor.

Die erfindungsgemäßen Polyhydantoine zeichnen sich durch besonders hohe Temperaturbeständigkeiten aus. Es werden z.B. bei der Prüfung nach DIN 46 453 eines mit diesen Polyhydantoinen beschichteten Kupferdrahtes teilweise

Erweichungstemperaturen von über 450°C festgestellt. Weiterhin zeigen die polymeren Reaktionsprodukte eine gute Löslichkeit und ausgezeichnete Verlaufseigenschaften bei der Beschichtung elektrischer Leiter und sind geeignet zur Verwendung als Kleber, Lacke, Folien, pulver, Fasern und Formkörper, wobei ihre Eigenschaften durch Zusatz von Füllstoffen, pigmenten und nieder- und/oder hochmolekularen Komponenten z.B. zur Herstellung von Lacken und Folien in weiten Grenzen variiert werden können.

Die mit den erfindungsgemäßen Polymeren modifizierten bekannten Polykondensate, wie z.B. Polyurethane, Polyamidimide, Polyester, Polyesterimide, Polyesteramidimide, Polyhydantoine, Polycarbonate, die zugemischt oder gegebenenfalls unter Mitverwendung von z.B. Polycarbonsäuren, deren Anhydriden, Estern, Polyolen usw. bei der erfindungsgemäßen Herstellung der beansPruchten Polyhydantoine an- und/oder einkondensiert werden, zeigen verbessertes TemPeraturverhalten und wesentlich verbesserte Verlaufseigenschaften, wenn derart modifizierte Produkte zur Beschichtung hitzeresistenter Substrate eingesetzt werden.

Die Mengenverhältnisse dieser Zusatzstoffe können je nach beabsichtigtem Verwendungszweck sehr unterschiedlich sein, vorzugsweise werden, bezogen auf das erfindungsgemäße Polyhydantoin, 5 - 500 Gew% eingesetzt.

Bevorzugt geeignet sind die erfindungsgemäßen Polyhydantoine für Einbrennlacke, insbesondere Draht- bzw. Elektroisolierlacke, wobei der Festgehalt der möglichen Lacklösungen, die auf den üblichen Lackiermaschinen sowie als Tränklacke verarbeitet werden, in weiten Grenzen schwanken kann und vorzugsweise im Bereich von 20 - 80 % liegt.

**Beispiel 1**

**A. Polydiphenylmethancarbodiimid**

Einer Lösung von 75 g (0,3 Mol) 4,4'-DiisocyanatodiPhenylmethan und 0,5 g Phenylisocyanat in 60 g Toluol und ß0 g Xylol werden bei RaumtemPeratur 0,5 g eines Gemisches aus 1-Methyl-1-PhosPha-2-cycloPenten-1-oxid und 1-Methyl-1-PhosPha-3-cycloPenten-1-oxid zugesetzt. Unter Gasuhranschluß wird innerhalb von ca. 2 1/2 Std. auf 65 - 70°C aufgeheizt, wobei eine hochviskose Polycarbodiimidsuspension mit den typischen IR-Banden bei 2100/2130 cm-1 erhalten und eine entwickelte $CO_2$Menge von 7 - 7,5 1 gemessen wird.

**B. Polyhydantoin**

Die Polycarbodiimidsuspension A wird wegen der erschwerten Rührfähigkeit möglichst schnell auf ca. 45°C abgekühlt und durch Zusatz von 100 g Phenol verdünnt. Bei 35°C werden zunächst 29,4 g Maleinsäureanhydrid (0,3 Mol), anschließend 115 g Phenol auf einmal eingetragen, und innerhalb von ca. 1/2 Std. erhöht man durch Nachheizen die Sumpftemperatur auf 80 - 85°C, wobei intensiv gerührt wird.

In 3 Std. wird unter Abdestillieren des Toluol/Xylol-Gemisches auf 1ß0 - 185°C aufgeheizt. Die Temperatur von 180 - 185 °C wird 4 Std. gehalten, wobei insgesamt ca. 149 g Destillat entfernt und 50 g Kresol im Verlauf des Viskositätsanstiegs zugesetzt werden. Man erhält eine klare rotbraune Polymerlösung mit einem Festgehalt von 34,1 % (5 Min. bei 360°C eingebrannt). Die Viskosität beträgt 3740 mPas (15 %ig mit Kresol verdünnt bei 20 °C in einem Höppler-Viskosimeter gemessen). Die für Hydantoinstrukturen typischen Banden werden in Lösung im IR bei 1750 - 1760, 1710 und 1430 (Ringbande) $cm^{-1}$ festgestellt. Durch Ausfällen aus Methanol wird der Polyhydantoinester als weißbraunes Pulver erhalten.

Im NMR ($CDC1_3$) zeigt das Polymere folgende Signale:

Aromatische Protonen: $\delta$ = 6,75 - 7,5 ppm (ca. 13 H)

$-\overset{|}{\underset{|}{C}}H$ : $\delta$ = 4,9 ppm (1 H)

$-Ø-CH_2-Ø-$ : $\delta$ = 3,95 ppm (2 H)

$-CH_2-\overset{O}{\overset{\|}{C}}-$ : $\delta$ = 3,2 ppm (2 H)

Mit der Elementaranalyse wird die aromatische Phenylester gruppe in 5-Stellung des Hydantoinringes bestätigt:

$(C_{24}H_{18}N_2O_4)_n$ $(398)_n$

| | ber: | gef: |
|---|---|---|
| C | 72,3 | 71,7 |
| H | 4,52 | 4,5 |
| N | 7,04 | 7,07 |

Die Beschichtung eines Kupferdrahtes (0,7 mm Durchmesser) mit der unter Beispiel 1 hergestellten Polyhydantoinlösung als ca. 22 %ige Lösung (Kresol/Xylol 1:1 verdünnt) auf einer Drahtlackiermaschine (Düsenauftragsverfahren) erfolgt mit ausgezeichneten Verlaufseigenschaften des Lackes. Bei Fahrgeschwindigkeiten von 8, 10 und 12 m/Min. und Einbrenntemperaturen von 400°C weist der Lackdraht Erweichungstemperaturen von über 470°C, Hitzeschockwerte von mindestens 260°C, Außenfaserdehnungen von 67 - 88 %, eine Schabefestigkeit von über 60 Doppelhüben, eine Filmhärte von mindestens 5 H und eine Durchschlagsfestigkeit von mindestens 8 KV und eine gute Chemikalienbeständigkeit auf.

Die Prüfung des Lackdrahtes erfolgt dabei nach DIN 46 453.

**Beispiel 2**

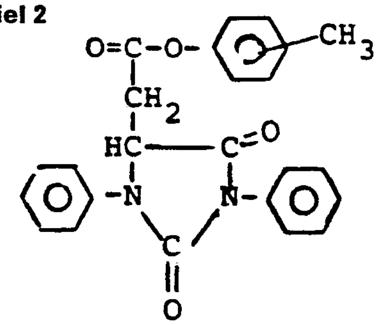

64,7 g einer 30 %igen Diphenylcarbodiimidlösung in o-Dichlorbenzol (IR-Banden der Carbodiimidgruppe bei 2100/2130 $cm^{-1}$) werden vorgelegt. Bei Raumtemperatur trägt man 9,8 g (0,1 Mol) Maleinsaureanhydrid

ein und in ca. 30 Min. werden bei 30 - 40°C 50 g Kresol unter Rühren zugegeben. Innerhalb von 3 Std. wird auf 180°C aufgeheizt, die 3 Std. gehalten werden.

Dann wird bei einer Sumptemperatur von 120 - 140°C unter Wasserstrahlpumpenvakuum ein Teil des Lösungsmittels entfernt und das Reaktionsprodukt als hellbrauner Feststoff durch Ausfällen aus einem Wasser/ n-propanol-Gemisch mit anschließender Trocknung isoliert das die für Hydantoinringe mit aromatischen Esterseitengruppen typischen IR-Banden bei 1765/1720/13901400 cm $^{1}$ mit der aromatischen Carbonylesterbande bei 1755 cm $^{1}$ aufweist. Zur weiteren Reinigung wird in Wasser/n-Propanol umgefällt, und NMR-spektroskopisch wird der Einbau des Kresols und somit die angenommene Struktur bestätigt.

Analyse: $C_{24}H_{20}N_2O_4$ (400)

| | C | H | N |
|---|---|---|---|
| ber. | 72,0 | 5,0 | 7,0 |
| gef. | 72,6 | 5,3 | 6,9 |

**Beispiel 3**

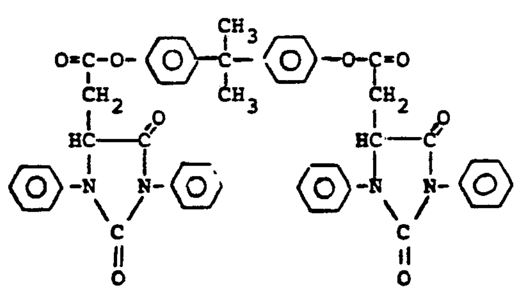

In 40 g Methylenchlorid und 80 g o-Dichlorbenzol werden bei Raumtemperatur 9,8 g (0,1 Mol) Maleinsäureanhydrid und 11,4 g (0,05 Mol) 2,2-Bis-(4-hydroxyphenyl)-propan eingetragen. Bei 40 - 45°C werden unter Rühren 80,8 g einer 24 %igen Diphenylcarbodiimidlösung in Toluol in 30 Min. mit 100 mg Tolyhydrochinon zugegeben. Unter Abdestillieren des Methylenchlorid/Toluol-Gemisches wird innerhalb von 2 Std. auf 120°C und nach Zugabe von 100 mg Diazabicyclooctan, 50 g ξ -Butyrolacton und 100 mg pToluolsulfonsäure in weiteren 2 Std. auf 180°C aufgeheizt. Es wird 2 Std. bei 180°C nachgerührt, ein Teil des Lösungsmittels unter Vakuum abdestilliert und das Reaktionsprodukt durch Ausfällen in Isopropanol/ $H_20$ isoliert. Nach dem Trocknen erhält man 21 g Reaktionsprodukt als graubraunes pulver, das die für Hydantoine mit aromatischen Esterseitengruppen in 5-Stellung charakteristischen IR-Banden bei 1765,1720, 1395-1400 cm$^{-1}$ und die aromatische Carbonylesterbande bei 1755 cm$^{-1}$ aufweist. Nach weiterem Umfällen in Isopropanol wird die angenommene Struktur durch das NMR-Spektrum bewiesen.

Analyse: $C_{49}H_{40}N_4O_8$ (812)

| | C | H | N |
|---|---|---|---|
| ber.: | 72,4 | 4,9 | 6,9 |
| gef.: | 72,9 | 4,6 | 6,8 |

**Beispiel 4**

Ein Blockpolycarbodiimid des Aufbaues -B-B-A-A-A-A-B-Baus 50 Mo1% poly-Diphenylmethancarbodiimid (A) und 50 Mo1% Poly-2,4 (80%)- und 2,6 (20%)-Tolylcarbodiimid (B) mit ca. 0,3 Mol Carbodiimidgruppen - hergestellt durch stufenweise Carbodiimidisierung von 37,5 g (0 15 Mol) des Diisocyanats von A und anschließend 32,4 g (0,15 Mol) des Diisocyanats von B durch Erhitzen in 150 g Methylenchlorid und 100 g Toluol in Gegenwart von 0,75 g Methylphospholinoxid unter Zusatz von 2,6 g (0,015 Mol) Tolylisocyanat als Regler - wird in dem angegebenen Lösungs-/Verdünnungsmittel vorgelegt.

Bei 40 - 45°C werden 100 g phenol und 50 g Kresol eingerührt und anschließend trägt man innerhalb von ca. 30 Min. 29,4 g (0,3 Mol) Maleinsäureanhydrid portionsweise bei 35 - 45°C ein. Innerhalb von 4 Std. wird unter Destillation auf 180°C aufgeheizt, wobei im Verlauf des Viskositätsanstiegs mit einem Lösungsmittelgemisch aus 60 g phenol und 60 g Kresol verdünnt wird. Die Reaktionstemperatur von 175 - 180°C wird 4 Std. gehalten, wobei insgesamt ca. 270 g Destillat anfallen. Man erhält eine braunrote polymerlösung mit einem Festgehalt von 32,3 % (5 Min. bei 360°C eingebrannt) und einer Viskosität von 1640 mPas ( 15 %ig mit Kresol verdünnt bei 20°C gemessen). Im IR werden die für Hydantoinringe mit aromatischen Esterseitengruppen charakteristischen Banden in phenolischen Lösungsmitteln bei 1750-1765, 1710 und 1415-1430 cm$^{-1}$ festgestellt.

Das Einbrennen von Proben dieser Polymerlösung auf Prüfblechen führt innerhalb von jeweils 10 Min. bei 200 bzw. 360°C zu braungelben, klaren elastischen Lackfilmen.

Nach der Beschichtung eines Cu-Drahtes (0,7 mm Durchmesser, Drahtlackiermaschine mit Vertikalprinzip, Ofenlänge 4 m) mit dieser ca. 24 %igen Polyhydantoinlösung (mit Kresol/Xylol verdünnt) werden bei 400°C Einbrenntemperatur und einem Vorschub von 8, 10 und 12 m/Min. Lackdrähte erhalten, die nach DIN 46 453 Erweichungstemperaturen von oberhalb 400°C und

Hitzeschockwerte von mindestens 260°C bei elektrischen Durchschlagswerten von 7-8 KV aufweisen, wobei sehr glatte Beschichtungen infolge der ausgezeichneten Verlaufseigenschaften des Lackes erzielt werden.

**Beispiel 5**

Ein Blockpolycarbodiimid mit der Struktur -D-D-D-D-$C_{-2}$ mit ca. 0,4 Mol Carbodiimid-Gruppen aus 20 Mol% PolyNaphthylencarbodiimid (C) und 80 Mol% Poly-Diphenylmethancarbodiimid (D) - hergestellt durch stufenweise Carbodiimidisierung von 17,1 g (0,08 Mol) des Diisocyanats von C und 80 g (0,32 Mol) des Diisocyanats von D unter Verwendung von 3,4 g (0,02 Mol) Naphthylisocyanat als Regler gegen Ende der Carbodiimidisierung in 200 g γ-Butyrolacton und 200 g Methylenchlorid - wird vorgelegt.

Bei 30-45°C werden 65 g (0,6 Mol) m-Kresol 70 und 39,2 g (0,4 Mol) Maleinsäureanhydrid in 100 g γ-Butyrolacton und 100 g N-Methylpyrrolidon unter intensivem Rühren eingetragen. Innerhalb von 3 Std. wird unter Abdestillieren des Methylenchlorids die Reaktionstemperatur auf 180°C gesteigert. Bei 175-185°C wird 4 Std. gerührt, wobei im Verlauf des Viskositätsanstiegs 50 g γ-Butyrolacton und 50 g Benzylalkohol eingetragen werden.

Man erhält eine braunrote umweltfreundliche nichtphenolische Polymerlösung mit einem Festgehalt von 26,8 % (5 Min. bei 360°C eingebrannt) und einer Viskosität von 1120 mPas (15 %ig mit N-Methylpyrrolidon verdünnt bei 20°C gemessen). Das aus Methanol ausgefällte Polymere zeigt die für Hydantoinringe mit aromatischen Esterseitengruppen typischen IR-Banden bei 1770, 1755, 1720 und 1405 $cm^{-1}$.

Die Applizierung der Polymerlösung auf einen Kupferdraht (∅ 0,7 mm) mit einer Drahtlackiermaschine (Ofenlänge 4 m) ergibt bei 400°C und 10-12 m/Min. Vorschub bei guten Verlaufseigenschaften elastische klare Beschichtungen mit Erweichungstemperaturen oberhalb von 400°C. Prüfung des Lackdrahtes nach DIN 46 453.

**Beispiel 6**

Eine Poly-Diphenylmethancarbodiimidlösung mit ca. 0,36 Mol Carbodiimidgruppen und den typischen IR-Banden bei 2140/2110 $cm^{-1}$ - hergestellt durch Carbodiimidisierung von 100 g (0,4 Mol) 4,4'-Diisocyanatodiphenylmethan unter Zusatz von 0,75 g Methylphospholinoxid, 7,5 g (0,08 Mol) Phenol als Regler in 100 g Methylenchlorid und 125 g Toluol durch Erhitzen auf 45-50°C bis zum Ende der Gasentwicklung - wird vorgelegt.

Bei 40°C werden 150 g Phenol eingerührt und innerhalb von 45 Min. werden bei 30-45°C 34,3 g (0,35 Mol) Maleinsäureanhydrid mit 50 g Phenol portionsweise eingetragen. Innerhalb von 2 Std. wird unter Abdestillieren des Toluol/Methylenchlorid-Gemisches auf 140°C aufgeheizt. Dann trägt man bei 120-125°C 7,7 g (0,04 Mol) Trimellithsäureanhydrid ein und erhöht die Sumpftemperatur innerhalb von 2 Std. auf 180°C, die 4 Std. gehalten werden, wobei insgesamt ca. 230 g Destillat entfernt und im Verlauf des Viskositätsanstiegs 100 g m-Kresol 70 und 20 g Phenol zugegeben werden. Nach dem Abkühlen auf 130°C werden 15 g Xylol eingerührt, und man erhält eine klare rotbraune Polymerlösung mit einem Feststoffgehalt von 35,5 % (5 Min. bei 360°C) und einer Viskosität von 1850 mPas (15 %ig mit m-Kresol 70 verdünnt bei 20°C). Durch den hohen Festgehalt wird der Einbau des Phenols als aromatische Ester-Seitengruppe der Hydantoinringe in das Polymermolekül bestätigt. Das durch Aus- und Umfällen in Methanol isolierte Polymere zeigt die charakteristischen IR-Banden bei 1765/1720/1390/-1400 $cm^{-1}$ und die aromatische Carbonylesterbande bei 1755 $cm^{-1}$.

Die mit dieser Lacklösung in ca. 24 %iger Konzentration (mit Kresol/Xylol 1 : 1 verdünnt) in einem 4-m-Ofen bei 8 und 10 m pro Minute beschichteten Cu-Drähte von 0,7 mm Durchmesser werden nach DIN 46 453 geprüft und besitzen Erweichungstemperaturen von mindestens 400°C, einen Hitzeschock von mindestens 260°C, gute Elastizitätswerte und gute Chemikalienbeständigkeit bei ausgezeichneten Verlaufseigenschaften des Lackes beim Applizieren.

**Beispiel 7**

61,8 g Poly-Diphenylmethancarbodiimid (0,3 Mol Carbodiimidgruppen) mit 1,75 g (0,015 Mol) Phenylcarbodiimid-Endgruppen in 100 g Methylenchlorid und 100 g Toluol werden vorgelegt.

Bei 35-40°C werden 200 g Phenol eingerührt. Anschließend werden innerhalb von 30 Min. 47,4 g (0,3 Mol) Acetoxybernsteinsäureanhydrid portionsweise zugegeben, wobei intensiv gerührt wird. Nach Zugabe von 200 mg Toluhydrochinon wird innerhalb von 5 Std. unter Destillation auf 180-185°C aufgeheizt. Bei dieser Temperatur wird ca. 3 Studen gerührt, wobei ca. 245 g Destillat erhalten und im Verlauf des Viskositätsanstiegs 80 g m-Kresol 70 zugesetzt werden. Im Destillat wird die durch Esterpyrolyse gebildete Essigsäure festgestellt. Der Festgehalt der braunroten klaren Polymerlösung beträgt 33,2 % (5 Min. bei 360°C), womit der Einbau des Phenols in das Polymermolekül bestätigt wird. Das NMR-Spektrum des aus Methanol aus- und umgefällten Produkts zeigt Übereinstimmung mit den unter Beispiel 1 angegebenen Daten. Die erhaltene Lacklösung weist eine Viskosität von 640 mPas (15 %ig mit Kresol verdünnt bei 20°C) und die für

aromatische Hydantoinester typischen IR-Banden bei 1750-1765, 1710 und 1415-1430 $cm^{-1}$ auf.

Das Einbrennen von Proben dieser Polymerlösung auf Prüfblechen bei 200, 300, 360° C führt zu klaren braunroten elastischen Polymerfilmen.

**Beispiel 8**

Eine Polycarbodiimidsuspension aus 75 Mo1% Polydiphenyl-methancarbodiimid und 25 Mo1% Poly-2,4 (80%)- und 2,6 (20%)-carbodiimid mit statistischer Verteilung der Struktureinheiten und mit ca. 0,4 Mol Carbodiimidgruppen - hergestellt durch Carbodiimidisierung einer Mischung aus 75 g (0,3 Mol) 4,4'-Diisocyanatodiphenylmethan und 17 4 g (0 1 Mol) eines Gemisches aus 2,4- und 2,6-Toluylendiisocyanat unter Zusatz von 0,5 g Methylphospholinoxid und 2,38 g (0,02 Mol) Phenylisocyanat als Regler in 100 g Methylenchlorid und 120 g Toluol wird vorgelegt.

Bei 30-40° C werden 150 g m-Kresol 70 unter intensivem Rühren eingetragen. Anschließend werden innerhalb Von 30 Min. 53,8 g (0,4 Mol) Chlorbernsteinsäureanhydrid in 100 g m-Kresol zugegeben. Nach Zugabe von 100 mg Toluhydrochinon wird unter Abdestillieren des Methylenchlorids und Toluols in 4 Std. auf 180° C aufgeheizt. Nach 6 stündigem Rühren, wobei im Zuge des Viskositätsanstiegs mit 50 g Phenol verdünnt wird, erhält man eine Polymerlösung mit den für Hydantoinringe typischen IR-Banden (1750-1770, 1410-1430 $cm^{-1}$) und einer Viskosität von 480 mPas (5 Min. bei 360° C eingebrannt) und einem Festgehalt von 36 %.

Die thermische Chlorwasserstoff-Eliminierung wird durch Nachschalten einer Waschflasche mit Aminlösung nachqewiesen, und das NMR-Spektrum des aus Methanol aus- und umgefällten Polymeren zeigt den Einbau des Kresols als aromatische Esterseitengruppe zum Hydantoinring, was durch den ermittelten Festgehalt von 36 % (5 Min. bei 360° C) bestätigt wird.

Bei 360° C eingebrannte Proben der Lacklösung ergeben klare elastische Polymerfilme.

**Beispiel 9**

In eine Carbodiimid-Lösung aus 250 g 4,4'-Diisocyanatodiphenylmethan, 6 g Phenylisocyanat und 1,5 g Methylphospholinoxid in 250 g Methylenchlorid und 250 g Toluol wird bei 55 °C unter Kühlung eine Lösung von 98 g Maleinsäureanhydrid in 188 g Phenol eingetragen. Dann werden 300 g Phenol zugegeben, die Temperatur auf 180° C gesteigert und dann 310 g Phenol/Kresol (1:1) zugegeben. Nach 3 Stunden bei 180° C erhält man eine 33 %ige Lösung des Polyhydantoinphenylesters mit einer Viskosität $\eta^{25}$ von 54000 mPas und den für Hydantoine charakteristischen Banden im IR-Spektrum bei 1705 und 1755 $cm^{-1}$.

An einem entsprechend Beispiel 1 lackierten Kupferdraht wird bei guten Verlaufeigenschaften eine maximale Außenfaserdehnung von 88 % und eine Erweichungstemperatur von 475° C gemessen.

**Patentansprüche**

1. Verfahren zur Herstellung von mindestens einen Hydantoinring enthaltenden Verbindungen, dadurch gekennzeichnet, daß man ein Carbodiimid der Formeln I oder II

$$R^1 - N = C = N - R^2 \quad (I) \qquad \left[ Y - N = C = N \right]_n \quad (II)$$

in denen

$R^1$ und $R^2$ gleich oder verschieden einen aliphatischen Rest mit 1 - 20 C-Atomen, einen cycloaliphatischen Rest mit 5 - 12 C-Atomen, einen aliphatisch-aromatischen Rest mit 6 - 20 C-Atomen, einen aromatischen Rest mit 6 - 16 C-Atomen, einen Heteroatome wie N, 0 oder S enthaltenden aromatischen oder cycloaliphatischen Rest mit 5 - 12 C-Atomen, die jeweils gegebenenfalls mit Halogen. Nitril-, $C_2$-$C_{12}$-Dialkyl-amino-, $C_7$-$C_{12}$-Alkylarylamino-, $C_2$-$C_{18}$-Alkoxycarbonyl-, $C_7$-$C_{18}$Aroxycarbonyl-, $C_2$-$C_{18}$-Alkylcarboxy-, $C_7$-$C_{18}$Arylcarboxy-, $C_1$-$C_{18}$-Alkoxy-, $C_6$-$C_{18}$-Aroxy-, $C_1$-$C_{18}$-Alkyl- bzw. Halogenalkyl- oder Nitrogruppen substituiert sein können, oder einen $C_2$-$C_{12}$-Dialkylamino-, $C_2$-$C_{10}$-Alkoxycarbonyl-, $C_6$-$C_{18}$-Glykosylrest oder eine -$Si(R^3)_3$-, -$Sn(R^3)_3$-, -SO $R^3$-Gruppe (mit $R^3$ = $C_6$-$C_{12}$-Aryl bzw. $C_1$-$C_8$-Alkyl) bedeuten oder miteinander als Glieder entsprechend cyclischer organischer Reste verknüpft sein können, und

Y die für $R^1$, $R^2$ angegebene Bedeutung hat und

n eine ganze Zahl von 2 - 2000 bedeutet, mit Derivaten des Bernsteinsäureanhydrids der Formel

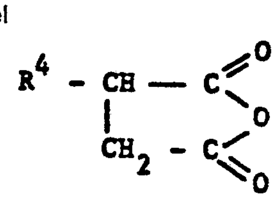

III ,

worin

$R^4$ ein Halogen, eine Alkylcarboxy- oder Arylcarboxygruppe bedeutet,

oder

mit Maleinsäureanhydrid in Gegenwart von aromatischen OH-funktionellen Verbindungen bei Temperaturen von ca. 20° C bis ca. 250° C, gegebenenfalls in einem Lösungsmittel und gegebenenfalls in Anwesenheit eines Katalysators umsetzt.

2. Verwendung der nach Anspruch 1 erhaltenen Polyhydantoine als hochtemperaturbeständige Überzugsmaterialien, Folien, Pulver, Kleber,

Lacke oder Formkörper.

## Claims

1. Process for the production of compounds containing at least one hydantoin ring, characterised in that a carbodiimide of the formulae I or II

$$R^1 - N = C = N - R^2 \quad \text{(I)} \qquad \left[ Y - N = C = N \right]_n \quad \text{(II)}$$

in which
$R^1$ and $R^2$ are identical or different and denote an aliphatic radical with 1 - 20 C atoms, a cycloaliphatic radical with 5 - 12 C atoms, an aliphatic-aromatic radical with 6 - 20 C atoms, an aromatic radical with 6 - 16 C atoms, an aromatic or cycloaliphatic radical which contains heteroatoms such as N, O or S and has 5 - 12 C atoms, each of which can optionally be substituted by halogen, nitrile,

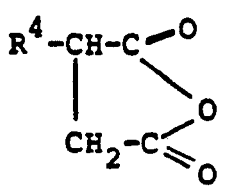

(III)

wherein
$R^4$ denotes a halogen, am aöluöcarboxy or arylcarboxy group,
or
with maleic acid anhydride in the presence of aromatic OH-functional compounds at temperatures of about 20°C to about 250°C, optionally in a solvent and optionally in the presence of a catalyst.

2. Use of the polyhydantoins obtained according to Claim 1 as high-temperature-resistant coating materials, films, powders, adhesives, lacquers or mouldings.

## Revendications

1. Procédé de préparation de composés contenant au moins un cycle hydantoïne caractérisé en ce qu'on fait réagir, en presence de composés aromatiques comportant des groupes fonctionnels OH à des températures d'environ 20°C à environ 250°C, éventuellement dans un solvant et éventuellement en présence d'un catalyseur, un carbodiimide répondant aux formules I ou II :

$$R^1 - N = C = N - R^2 \quad \text{(I)} \qquad \left[ Y - N = C = N \right]_n \quad \text{(II)}$$

dans lesquelles
$R^1$ et $R^2$, identiques ou différents, représentent un reste aliphatique ayant 1 à 20 atomes de carbone, un reste cycloaliphatique ayant 5 à 12 atomes de carbone, un reste aliphatique-aromatique ayant 6 à 20 atomes de carbone, un reste aromatique ayant 6 à 16 atomes de carbone, un reste aromatique ou cycloaromatique ayant 5 à 12 atomes de carbone, contenant un hétéroatome comme N, O ou S (qui peuvent éventuellement être chacun substitués par de l'halogène, des groupes nitrile, dialkyl-amino en $C_2$ $C_{12}$, alkylarylamino en $C_7$-$C_{12}$, alcoxycarbonyle en $C_2$-$C_{18}$, aroxycarbonyle en $C_7$-$C_{18}$, alkylcarboxyle en $C_2$-$C_{18}$, arylcarboxyle en $C_7$-$C_{18}$, alcoxy en $C_1$-$C_{18}$, aroxy en $C_6$-$C_{18}$, alkyle ou halogènoalkyle en $C_1$-$C_{18}$ ou mitro), ou un reste dialkylamino en $C_2$ $C_{12}$, alcoxycarbonyle en $C_2$-$C_{10}$, glycosyle en $C_6$-$C_{18}$ ou un groupe -$Si(R^3)_3$, -$Sn(R^3)'$, -$SO_2R_3$ (où $R^3$ représente un groupe aryle en $C_6$-$C_{12}$ ou alkyle en $C_1$-$C_8$) ou bien ils peuvent être reliés l'un à l'autre pour former des chaînons de restes organiques cycliques correspondants, et
Y a le sens indiqué pour $R^1$, $R^2$, et
n est un nombre entier valant 2 - 2000,
avec des dérivés de l'anhydride de l'acide succinique de formule:

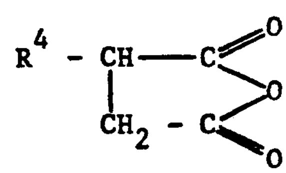

, (III)

dans laquelle
$R^4$ représente un halogène, un groupe alkylcarboxyle ou arylcarboxyle,
ou
avec l'anhydride de l'acide maléique.

2. Utilisation des polyhydantoïnes, obtenues selon la revendication 1, comme matières de revêtement, feuilles, poudres, adhésifs, vernis ou objets moulés, stables aux températures élevées.